# EUROPEAN PATENT APPLICATION

(11) **EP 2 016 842 A1**
(43) Date of publication of application: **21.01.2009**
(21) Application number: 07014266.6
(22) Date of filing: 20.07.2007
(51) Int. Cl.: A41D 13/00

(54) **Heat transferring garment**

(71) Applicant: Tiesnitsch, Johannes Ijsbrand, 6001 EX Weert (NL)
(72) Inventor: Tiesnitsch, Johannes Ijsbrand, 6001 EX Weert (NL)

(57) **Abstract**

Heat transferring garment, comprising an outer layer, an inner layer and a network of tubes, wherein the inner layer is locally connected to the outer layer along lines having a mutual distance and the tubes run between the inner and the outer layer between pairs of neighboring lines. Preferably the lines run in a direction essentially parallel to a length direction of a body part or a limb the garment is intended to cover and also preferably the inner and outer layers are of stretchable material.

## Description

The invention relates to a heat-transferring garment, comprising an outer layer, an inner layer and a network of tubes.

Such garment is known from Patent Application FR 2,836,339. This document seeks to provide a garment that can be laundered without damaging the tubes, that can be easily maintained and that allows evaporation of transpiration fluids to the environment. The document teaches to stitch the tubes to the outer layer and increasing the stitch density with the bend radius of the tube.

A disadvantage of the known garment is the tight connection of the tubes to the garment, which impedes easy replacement of tubes and which makes the garment little flexible to the detriment of wearing comfort.

Aim of the invention is to provide a heat-transferring garment that can be easily maintained, gives good wearing comfort and allows easy laundering without damaging the tubes.

This aim is achieved in the inner layer is connected to the outer layer along lines having a mutual distance and the tubes run between pairs of neighboring lines and over at least part of their length between the inner and the outer layer.

Thus the tubes are kept in place without being stitched or otherwise fixed to the outer or inner layer. The tubes can be easily replaced and removed before laundering the garment.

The construction according to the invention also avoids the risk of tubes from being punctured during sewing the tubes on the outer layer as in the known garment.

The garment comprises an outer layer. Suitable materials for outer layers for heat-transferring garment are known per se in the art. Preferably the material is breathable to allow evaporation of transpiration fluids from the body of the person wearing the garment to the environment.

Tubes, suitable for use in heat-transferring garment are known per se in the art. The tubes are present in the garment close to the body of the person wearing it to allow optimal heat exchange from the body to the cooling or heating liquid flowing through the tubes. The tubes are flexible and have good heat conductivity properties.

The tubes in the garment when in use will be connected to a device supplying warm or cold fluid to circulate through the tubes thus removing or supplying heat from or to the body.

The lines may run parallel or form and angle but should not intersect in the garment. Thus two consecutive lines form a space, bordered by the parts of the inner and outer layer between the two lines and open-ended at both sides. Through this space the tube can be entered. When the tube reaches an open end it can be bent back and entered into a neighboring space where it runs in a direction opposite to that in the former channel. In this way the tubes can be positioned in consecutive spaces in a back and forth manner covering the whole area of the garment. Preferably a number of tubes are used, each running through a limited number of spaces and at its ends connected to a next tube or to a device providing a heat transfer fluid. This allows easy applying and removing of the tubes in smaller parts rather than as a whole. This enhances maintainability and comfort of use, e,g, when the garment needs laundering or when a damaged or leak tube has to be replaced.

The layers can be connected e.g. by stitching or sewing these together along the desired lines. The distance of consecutive stitches may vary broadly. Practical distances may range from 0.1 mm to 10 cm, though more preferable between 0,5 and 5 cm. Distance between two consecutive lines depends on the size, in particular the diameter of the tube to be applied in the space formed. Tubes can be present between each pair of neighboring lines. It is also possible to pass over a space and use the next one. Tube density, i.e. the number of tubes per unit of area, may vary over the garment and can be lower e.g. at places where more flexibility or stretch is required to allow unhampered body movement.

To diminish unwanted and useless heat exchange of the tubes with the environment any thermally isolating and/or absorbing material can be present between the inner and the outer layer between neighboring lines in the spaces where the tubes run.

In the garment according to the invention the tubes can run in virtually any pattern but preferably the lines, and as a consequence the tubes between the lines, run in a direction essentially parallel to a length direction of a body part or a limb the garment is intended to cover.

In the body of a person moving, e.g. running or cycling, e.g. the chest will expand through breathing, arm and leg muscles will locally swell. This requires the garment to be stretchable in a direction perpendicular to the length direction of the chest or the limbs. Therefore also preferably the inner and outer layers are of stretchable material. More preferably the inner and outer layer have comparable stretch properties.

Thus the stretchable inner and outer layers due to their stretchable nature can match required stretching due to the local body expansion and movement, whereas the, usually not stretchable tubes are not. This greatly enhances comfort of the wearer. The means connecting the inner and outer layer may but need not provide flexibility and stretchability in the direction of the lines. When stitched together the thread used may be stretchable. Preferably this thread has limited stretchability thus favoring stretchability of the garment perpendicular to the lines.

The inner layer material preferably is thin and allows easy evaporation of transpiration fluid. It will also be sufficiently flexible to allow free wearer movement. This type of material is known in the art and does not form part of the invention.

In an embodiment of the invention the inner layer comprises a row of holes between at least the neighboring pairs of lines between which tubes run.

At the position of the holes the tubes are directly exposed to the wearer's body when in use, enhancing the heat exchange efficiency.

This effect can even be increased when the tubes are woven through the holes, the tubes between two consecutive holes running alternating between the inner and the outer layer and along a side of the inner layer not facing the outer layer. The shape of the holes is not critical and can be e.g. circular, oval or rectangular.

In this embodiment the holes function as spaces through which the tubes can run as being woven from one side of the inner layer to the other. Thus the tube runs alternating between the outer and the inner layer and along the side of the inner layer facing the wearer's body when in use, i.e. the side not facing the outer layer. The tubes then are over more than half their length directly exposed to the wearer and there they are not screened from the body by the inner layer. This provides optimized heat transfer. The tubes thus form a unity with the inner layer but also in this embodiment can be easily applied or removed. Also here one tube can be used but preferably a number of connected tubes is used as described above.

The holes can be present in various patterns, thus allowing more flexibility in the track formed by the tubes between the neighboring lines. The possibility of varying the tube density, distance or direction allows applying the tubes optimally adapted to the body shape and local differences in required mobility of the garment when in use.

The inner layer will preferably also be connected to the outer layer along the edges of the garment (top and bottom collar, belt, sleeves) to make them a unity. It is moreover also possible to connect the inner and the outer layers locally at other places when it is desired to restrict mutual mobility of the layers.

The size and number of the holes is not critical and can be chosen in connection with the dimensions of the tube applied and the tube density and track envisaged.

In another embodiment the inner layer is a net having meshes

This embodiment brings optimal exposure of the tubes to the wearer's body and best heat transfer. The mesh size of the net will be chosen in connection with the tube dimensions. The inner layer net preferably is also connected to the outer layer along the edges of the garment (e.g. top and bottom collar, belt, sleeves) to make them a unity. It is also possible to connect the inner and the outer layers locally at other places when it is desired to restrict mutual mobility of the layers.

The tubes may be woven through meshes of the net.

The invention encompasses any types of garment; most advantageously the garment is a vest, a jacket, shorts or trousers but it may also be a glove, a sock, a cap or an arm or leg piece.

The invention will be elucidated by the following exemplary drawings, without being restricted to the shown specific embodiments.

In these drawings:
Fig. 1 is a view on the inside of the back part of a garment having an integral inner layer.
Fig. 2 is a view on the inside of the back part of a garment having an inner layer comprising holes; and
Fig. 3 is a view on the inside of the back part of a having a net as inner layer.

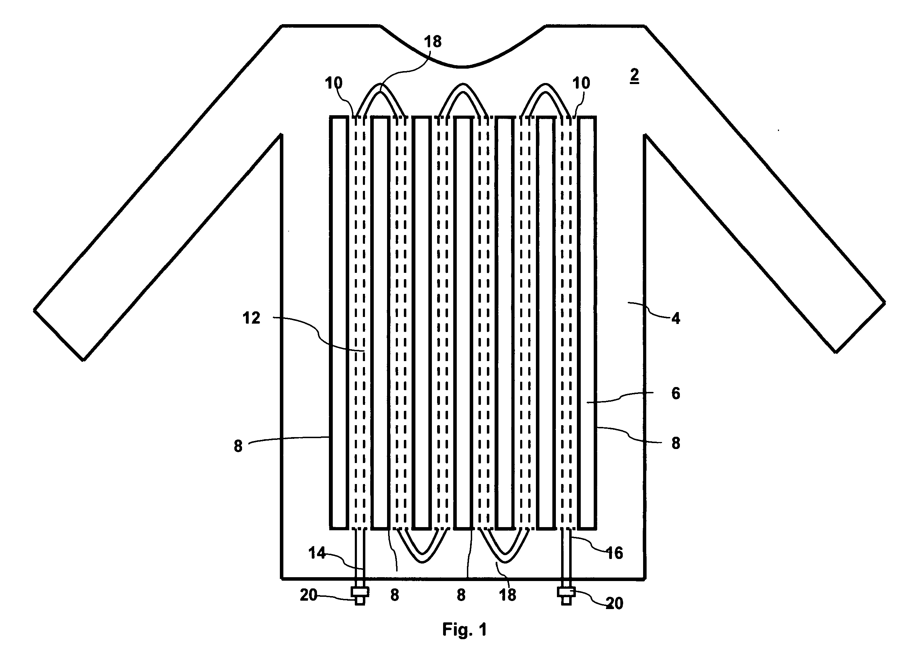

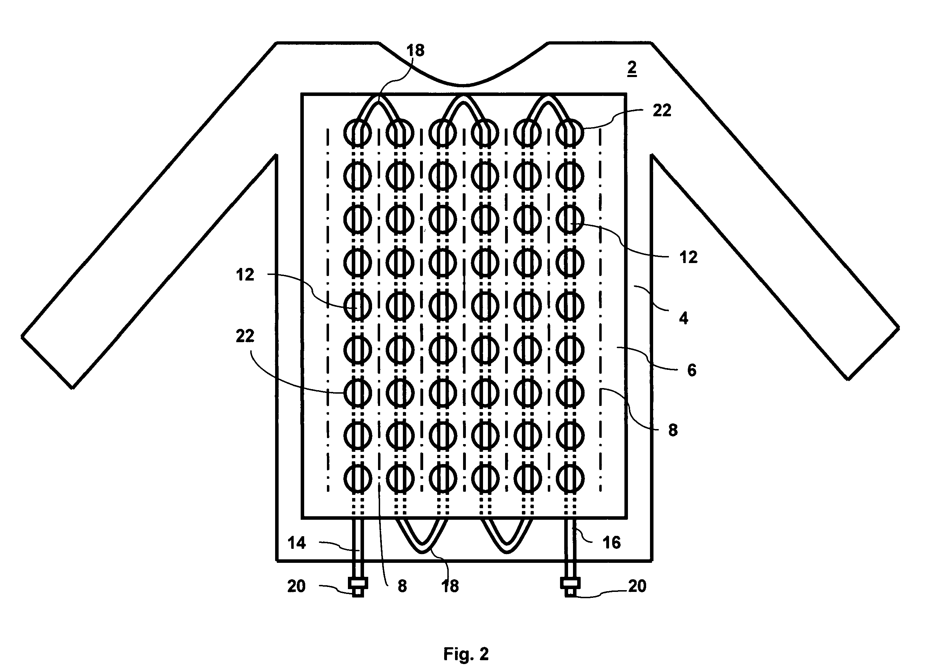

In Fig. 1, 2 is a heat transfer garment comprising an outer layer 4 and an inner layer 6. Inner layer 6 is stitched to outer layer 4 along lines 8. The stitched lines 8 form open-ended spaces 10 between outer layer 4 and inner layer 6 and neighboring pairs of lines. Through spaces 10 tube 12 runs from first tube end 14 to second tube end 16. The tube track inside spaces 10 is shown as dashed lines. Via curves 18 tube 12 runs from one channel to a neighboring channel. Ends 14 and 16 are provided with a connector 20 for connecting tube 12 to a device supplying heat-transfer fluid.

In Fig. 2 same numbers as in Fig.1 denote the same items. Stitched lines 8 are shown dash-dotted. Tube 12 now runs through holes 22 alternating between inner layer 6 and outer layer 4 (dashed parts) and on the visible side of the inner layer 6.

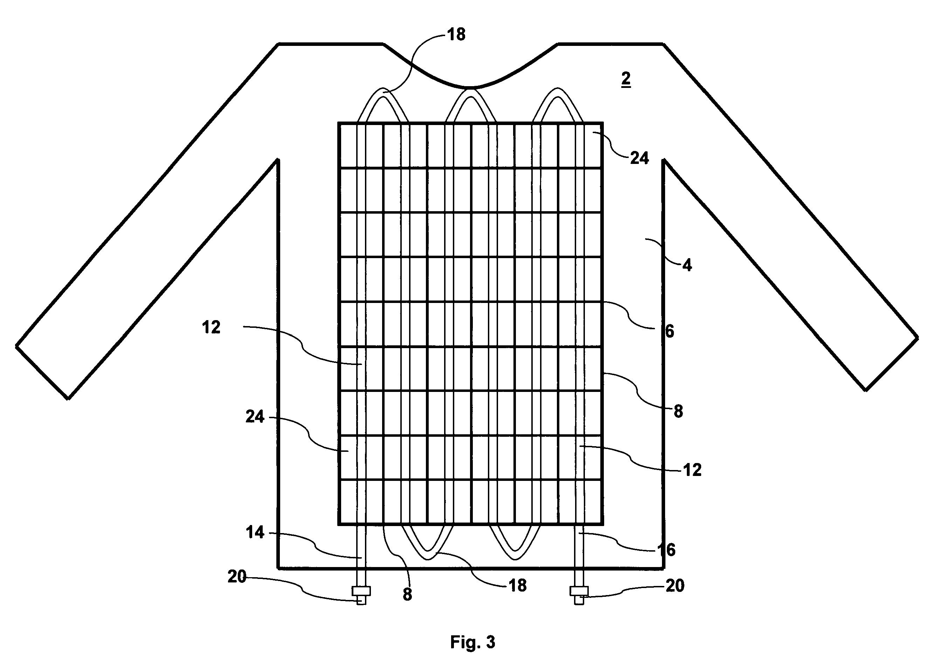

In Fig. 3 again same numbers denote same items as in Figs. 1 and 2. Stitched lines 8 coincide with vertical mesh boundaries. Tube 12 now runs woven through meshes 24. In this embodiment tube 12 is over virtually its total length uncovered by inner layer 6 and when the garment is in use it will be over virtually its total length in direct contact with the wearer thus providing optimal heat exchange.

## Claims

1. Heat transferring garment, comprising an outer layer, an inner layer and a network of tubes, wherein the inner layer is connected to the outer layer along lines having a mutual distance and the tubes run between pairs of neighboring lines and over at least part of their length between the inner and the outer layer.

2. Garment according to claim 1, wherein the lines run in a direction essentially parallel to a length direction of a body part or a limb the garment is intended to cover.

3. Garment according to claim 1 or 2, wherein the inner and outer layers are of stretchable material.

4. Garment according to any of claims 1-3, wherein the inner layer comprises a row of holes between at least the neighboring pairs of lines between which tubes run.

5. Garment according to claim 4, wherein the tubes are woven through the holes, the tubes between two consecutive holes running alternating between the inner and the outer layer and along a side of the inner layer not facing the outer layer.

6. Garment according to any of claims 1-3, wherein the inner layer is a net having meshes.

7. Garment according to any of claims 1-6, the garment being a vest, a jacket, shorts or trousers.
